(19) Europäisches Patentamt
European Patent Office
Office européen des brevets

(11) **EP 3 552 650 B1**

(12) **EUROPÄISCHE PATENTSCHRIFT**

(45) Veröffentlichungstag und Bekanntmachung des Hinweises auf die Patenterteilung:
**23.06.2021   Patentblatt 2021/25**

(51) Int Cl.:
***A61M 16/06*** *(2006.01)*

(21) Anmeldenummer: **19168981.9**

(22) Anmeldetag: **12.04.2019**

(54) **MASKENWULST FÜR EIN PATIENTENINTERFACE**

MASK BEAD FOR A PATIENT INTERFACE

JUPE DE MASQUE POUR UNE INTERFACE PATIENT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **12.04.2018   DE 102018002998**

(43) Veröffentlichungstag der Anmeldung:
**16.10.2019   Patentblatt 2019/42**

(73) Patentinhaber: **Löwenstein Medical Technology S.A.**
**2557 Luxembourg (LU)**

(72) Erfinder: **Eifler, Martin**
**25348 Glückstadt (DE)**

(74) Vertreter: **Marx, Thomas**
**Löwenstein Medical Technology GmbH + Co. KG**
**IP Management**
**Kronsaalsweg 40**
**22525 Hamburg (DE)**

(56) Entgegenhaltungen:
EP-A2- 1 982 740          WO-A1-01/62326
WO-A1-2014/183167       WO-A1-2015/020535
WO-A1-2016/032343       WO-A1-2017/049360
DE-A1-102007 057 091    DE-A1-102013 019 149
US-A1- 2002 148 472     US-A1- 2012 138 062
US-A1- 2014 174 446     US-A1- 2014 283 842

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Masken werden im Bereich der Beatmung und der Schlaftherapie eingesetzt, wo ein Patient mittels einer Flow- oder Druckquelle mit Atemgas versorgt wird. Eine Maske stellt die Schnittstelle zwischen Patient und Gerät dar. Sie besteht meistens aus einem Maskenkörper mit einem Maskenwulst und wird mittels einer Bänderung und eventuell mit einer zusätzlichen Stirnabstützung am Kopf des Patienten fixiert. Der Maskenkörper besteht in der Regel aus einem relativ steifen Kunststoff und der Maskenwulst, der am Gesicht, bzw. an Gesichtspartien des Patienten anliegt, bestehend aus einem relativ dünnen, weichen Material, vorzugsweise aus einem hautfreundlichen Silikon, einem thermoplastischen Elastomer (TPE) oder aus Polyurethan und besitzt in der Regel eine Dichtlippe, um eine ausreichende Abdichtung zu gewährleisten und Leckagen zu verhindern.

Die Funktion eines Maskenwulstes besteht unter anderem darin, das Gewicht des Maskenkörpers sanft gegen das Gesicht abzustützen. Hierfür weist der Maskenwulst eine Festigkeit auf, die es erlaubt, den Maskenkörper abzustützen und dennoch, durch das relativ weiche Material des Maskenwulstes, die empfindlichen Gesichtspartien des Patienten zu schützen.

Wie eingehend beschrieben werden herkömmliche Maskenwülste aus einem relativ dünnen, weichen Material mit einer geringeren Festigkeit gebildet, um sowohl einem Patienten beim Tragen einer entsprechenden Maske einen höchstmöglichen Komfort bereitzustellen als auch eine hohe Anpassungsfähigkeit des Maskenwulstes an die Gesichtskontur des Patienten zu erzielen.

Die WO 2001/062326 A1 offenbart einen Maskenwulst, welcher mondsichelartig geformte Zonen höherer Tragfähigkeit aufweist, welche in Kombination mit Zonen geringerer Tragfähigkeit eine gerichtete Bewegung des Maskenwulstes begünstigt und dabei eine ungleichmäßige Stützung des Maskenwulstes bewirkt.

Die US 2002/0148472 A1 offenbart einen Maskenwulst, welcher zwischen dem Auflagebereich und dem Maskenwulstanschluss Rippen-förmige Stützstrukturen aufweist.

Diese Rippen geben dem Maskenwulst eine Stützfunktion im Bereich der Rippen und leiten die Kräfte, z.B. durch den Anpressdruck beim Festschnallen der Maske, linear auf den Auflagebereich. Je nach Anordnung und Anzahl der Rippen werden die Kräfte unter anderem in Gesichtsbereiche geführt, welche dem zusätzlichen Druck gegenüber empfindlich sind und sich so ein geringer Tragekomfort einstellt. Im Bereich der Rippen ist der Maskenwulst zusätzlich derart versteift, dass eine Anpassbarkeit an die Gesichtskontur gehemmt ist.

Die aus dem Stand der Technik bekannten Lösungen weisen jedoch den Nachteil auf, dass die Verringerung der Festigkeit des Materials des Maskenwulstes auch zu einer Verringerung der Abstützfunktion des Maskenwulstes führt. Somit können zwar durch die Verringerung der Festigkeit des Materials der Komfort und die Anpassbarkeit des Maskenwulstes an die Gesichtskontur des Patienten verbessert werden, gleichzeitig verschlechtert eine Verringerung der Festigkeit jedoch auch die Abstützfunktion des Maskenwulstes. Die aus dem Stand der Technik bekannten Lösungen, welche die Abstützfunktion unterstützen, weisen den Nachteil einer Verringerung der Anpassbarkeit und des Tragekomforts auf.

Es ist daher Aufgabe der vorliegenden Erfindung, einen Maskenwulst bereitstellen, der die Kraft auf definierte Punkte des Maskenwulsts leitet und dem gesamten Maskenwulst gleichzeitig eine hohe Stabilität gibt und dabei eine hohe Anpassbarkeit des Maskenwulstes bietet.

Diese Aufgabe wird durch einen Maskenwulst gemäß dem erfindungsgemäßen Anspruch 1 gelöst.

Erfindungsgemäß wird die Aufgabe gelöst durch einen Maskenwulst für ein Patienteninterface bestehend aus einem Nasenrückenbereich, mindestens einem Seitenbereich, einem Basisbereich, einem Maskenwulstanschluss und einem Auflagebereich zum Patienten, wobei der Auflagebereich sich ausgehend von den Seitenbereichen und dem Basisbereich sowie dem Nasenrückenbereich zur Öffnung hin erstreckt, wobei der Auflagebereich als Dichtlippe ausgeführt ist, welche die zentrale Öffnung umrandet, die zumindest der Einführung einer Nase des Patienten dient, wobei der Maskenwulst aus mindestens einem elastischen Material ausgebildet ist und mindestens eine Stützstruktur umfasst, wobei die Stützstruktur in dem Maskenwulst bereichsweise zum Auflagebereich weisend und bereichsweise zum Maskenwulstanschluss weisend ausgebildet ist, wobei die Stützstruktur mindestens zwei Verstärkungspunkte und mindestens eine Verbindungsstruktur zwischen zwei Verstärkungspunkten aufweist, wobei die mindestens eine Verbindungsstruktur als Bogen ausgebildet ist, wobei der Bogen von dem Auflagebereich fortweisend ausgebildet ist, und die Verstärkungspunkte am Übergang des Nasenrückenbereichs zum Seitenbereich ausgebildet sind.

Gegenstand der vorliegenden Erfindung ist ein Maskenwulst für ein Patienteninterface bestehend aus einem Nasenrückenbereich, mindestens einem Seitenbereich, einem Basisbereich, einem Maskenwulstanschluss und einem Auflagebereich zum Patienten, wobei der Auflagebereich sich ausgehend von den Seitenbereichen und dem Basisbereich sowie dem Nasenrückenbereich zur Öffnung hin erstreckt, wobei der Auflagebereich als Dichtlippe ausgeführt ist, welche die zentrale Öffnung umrandet, die zumindest der Einführung einer Nase des Patienten dient. Der erfindungsgemäßen Maskenwulst ist sowohl für Nasalmasken als auch für Fullfacemasken einsetzbar.

Der Maskenwulst kann in die Bereiche Nasenrückenbereich, Seitenbereiche und Basisbereich eingeteilt werden.

Als Nasenrückenbereich wird der Bereich des Maskenwulstes bezeichnet, der eine Wölbung aufweist, die einen Winkel >180° aufweist. Der Nasenrückenbereich weist typischerweise eine Wandstärke von 0,2 - 0,7 mm auf. Der Nasenrückenbereich erstreckt sich in der Regel von dem Auflagebereich in Richtung des Maskenwulstanschlusses.

Als Basisbereich wird ein Bereich des Maskenwulstes bezeichnet, der an einer, dem Nasenrückenbereich gegenüberliegenden, Seite des Maskenwulstes ausgebildet ist und eine im Wesentlichen lineare Ausprägung aufweist. Beispielsweise weist der Basisbereich eine Wandstärke von 0,5 - 1,2 mm auf. Der Basisbereich erstreckt sich in der Regel von dem Auflagebereich in Richtung des Maskenwulstanschlusses.

Als Seitenbereich wird jeweils ein Bereich des Maskenwulstes bezeichnet, der sich auf jeder Seite der Öffnung des Maskenwulstes zwischen dem Nasenrückenbereich und dem Basisbereich erstreckt, wobei die beiden Seitenbereiche zueinander eine zulaufende bzw. konische Form aufweisen. Beispielsweise weist der Seitenbereich eine Wandstärke von 1,2 - 2,5 mm auf. Die Seitenbereiche erstrecken sich jeweils von dem Auflagebereich in Richtung des Maskenwulstanschlusses. Optional kann der Maskenwulst eine Doppellippenstruktur aufweisen.

Der Nasenrückenbereich und der Basisbereich grenzen jeweils an die Seitenbereiche an, wobei zwischen Nasenrückenbereich und Seitenbereichen und Basisbereich und Seitenbereichen jeweils Übergangsbereiche ausgebildet sind. Ein Übergangsbereich ist dabei ein Bereich, in dem sich der Verlauf der Form des Maskenwulstes ändert. Der Übergangsbereich zwischen Nasenrückenbereich und dem jeweiligen angrenzenden Seitenbereich ist der Bereich, in dem der Winkel des Maskenwulstes von einer gebogenen Form, die einen Winkel von über 180° aufweist, in eine lineare Form übergeht. Der Übergangsbereich zwischen Basisbereich und Seitenbereich ist jeweils der Bereich, in dem der Maskenwulst von einer konisch zulaufenden Form in einem Winkel von über 90° in einen linearen Verlauf übergeht.

Erfindungsgemäß ist der Maskenwulst aus mindestens einem elastischen Material ausgebildet und umfasst mindestens eine Stützstruktur, wobei die Stützstruktur in dem Maskenwulst bereichsweise zum Auflagebereich weisend und bereichsweise zum Maskenwulstanschluss weisend ausgebildet ist.

Dabei kann die Stützstruktur in ihrem Verlauf unterschiedliche Abstände zum Auflagebereich aufweisen. Beispielsweise kann die Stützstruktur an einem Scheitelpunkt des Nasenrückenbereichs einen größeren Abstand zum Auflagebereich aufweisen als an einem seitlichen Bereich des Nasenrückenbereichs. Auch kann die Stützstruktur in mindestens einem Seitenbereich des Maskenwulstes mindestens zwei verschiedene Abstände zum Auflagebereich aufweisen. Beispielsweise kann die Stützstruktur in einem mittleren Bereich eines Seitenbereichs einen größeren Abstand zum Auflagebereich aufweisen als an den Übergangspunkten zum Nasenrückenbereich oder zum Basisbereich. Der Auflagebereich kann abweichend von dem Nasenrückenbereich, den Seitenbereichen oder dem Basisbereich beispielsweise eine Wandstärke im Bereich von 0,2 - 0,75 mm aufweisen. Alternativ weist der Auflagebereich die gleiche Wandstärke auf, wie der Nasenrückenbereich, die Seitenbereiche oder der Basisbereich.

Der Maskenwulst ist aus einem hautfreundlichen Material, bevorzugt aus Silikon in einem Shore-Härtebereich zwischen 35 bis 65 Shore A, bevorzugt 40-60 Shore A, ausgebildet.

Der Maskenwulst kann einteilig hergestellt werden oder in einem Mehrkomponenten-Spritzgussverfahren, beispielsweise im 2K-Verfahren, hergestellt sein. Bei der Herstellung im 2K-Verfahren wird der Maskenwulst aus Materialien mit verschiedenen Härtegraden hergestellt, dabei wird beispielsweise ein weicheres Material an ein härteres Material angespritzt. Durch die Herstellung im Mehrkomponentenverfahren kann ein Maskenwulst mit unterschiedlichen Materialeigenschaften hergestellt werden, wodurch ein Maskenwulst herstellbar ist, der hinreichend steif und trotzdem nachgiebig ist. Beispielsweise ist die Stützstruktur in einem 2K-Verfahren herstellbar. Der Maskenwulst kann aus mindestens zwei, beispielsweise fünf verschiedenen Materialstärken ausgebildet sein. Der Kontakt der unterschiedlichen Komponenten wird bevorzugt über eine Haftung der Materialien erreicht, alternativ oder zusätzlich können mechanische Kontaktstellen die Haftung verstärken (Formschluss).

Die Stützstruktur ist in der Regel in dem Maskenwulst integral ausgebildet. Alternativ kann die Stützstruktur als ein auf eine Innen- oder Außenwand des Maskenwulstes aufgebrachtes Element ausgebildet sein. Die Stützstruktur ist in der Regel ausgebildet, eine Wandstärke aufzuweisen, die sich von der Wandstärke des umgebenden Maskenwulstes bzw. der einzelnen Bereiche (Nasenrückenbereich, Seitenbereiche, Basisbereich), unterscheidet. Insbesondere weist die Stützstruktur eine Wandstärke auf, die in einem Bereich zwischen $\frac{1}{12}$ und $\frac{1}{8}$, insbesondere $\frac{1}{10}$, stärker ausgebildet ist, als die Wandstärke des umgebenden Maskenwulstes bzw. der entsprechenden Bereiche. Beispielsweise kann die Stützstruktur bei einer $\frac{1}{10}$ stärkeren Ausgestaltung im Vergleich zum umgebenden Material im Bereich des Nasenrückenbereichs des Maskenwulstes eine Wandstärke im Bereich zwischen 0,33 - 0,77 mm aufweisen.

Typischerweise ist die Stützstruktur vollständig gefüllt ausgebildet. In einer alternativen Ausführungsform kann die Stützstruktur zumindest teilweise gefüllt ausgebildet sein. Die Stützstruktur kann eine homogen gefüllte Struktur aufweisen. Alternativ kann die Stützstruktur eine interne Formgebung aufweisen, die die Stützfunktion der Stützstruktur unterstützt. Optional kann die Stützstruktur als Dichtstruktur ausgebildet sein, die eingerichtet ist, den Maskenwulst, an dem Auflagebereich gegenüber einem Gesicht eines Patienten abzudichten.

In einer Weiterbildung der Erfindung umgibt die Stützstruktur die Öffnung zumindest zu zwei Drittel oder vollständig. Bei einer Ausgestaltung, bei der die Stützstruktur zumindest zu zwei Drittel die Öffnung des Maskenwulstes umgibt, weisen in der Regel der Nasenrückenbereich und die Seitenbereiche des Maskenwulstes die Stützstruktur auf, während der Basisbereich des Maskenwulstes stützstrukturfrei ausgebildet ist. Durch die Umrandung der Öffnung des Maskenwulstes mit der Stützstruktur zumindest zu zwei Drittel, kann der Maskenwulst optimal abgestützt werden. Bei der vollständigen Umrandung der Öffnung des Maskenwulstes mit der Stützstruktur ist die Stützstruktur zudem in dem Basisbereich ausgebildet. Die vollständige Umrandung liefert eine besonders gleichmäßige Verteilungsmöglichkeit von Gewicht auf Gesichtsbereiche des Patienten.

In Ausgestaltung weist die Stützstruktur mindestens zwei Verstärkungspunkte und mindestens eine Verbindungsstruktur zwischen zwei Verstärkungspunkten auf. In der Regel ist die Stützstruktur durchgängig ausgebildet, wobei die Stützstruktur mindestens zwei Verstärkungspunkte und mindestens eine die beiden Verstärkungspunkte verbindende Verbindungstruktur umfasst. Durch die Ausbildung der Stützstruktur wird die Festigkeit des Maskenwulstes verstärkt. Die Verbindungstruktur kann linear oder gebogen ausgebildet sein. Alternativ kann die Verbindungstruktur eine gewellte, gezackte oder geschwungene Struktur aufweisen.

Die Stützstruktur kann eine durchgehend gleiche Wandstärke aufweisen. In der Regel weist die Stützstruktur in den einzelnen Bereichen unterschiedliche Wandstärken auf. In der Regel weist die Stützstruktur jeweils eine $\frac{1}{12}$ bis $\frac{1}{8}$, insbesondere eine $\frac{1}{10}$ stärkere Wandstärke auf, als der jeweilige Bereich, in dem sie ausgebildet ist.

In der Regel weist die Stützstruktur zudem eine durchgehend gleiche Breite auf. Die Breite der Stützstruktur ist der Querschnitt der Stützstruktur des Maskenwulstes in angelegtem Zustand in horizontaler Richtung. Optional kann die Breite der Stützstruktur unterschiedlich ausgebildet sein. Beispielsweise kann die Breite der Stützstruktur im Bereich mindestens eines Verstärkungspunktes verringert ausgebildet sein.

In einer weiteren Ausgestaltung ist die mindestens eine Verbindungsstruktur als Bogen ausgebildet, wobei der Bogen von dem Auflagebereich fortweisend ausgebildet ist. Der Bogen erstreckt sich dabei beispielsweise von einem Verstärkungspunkt über den Nasenrückenbereich zu einem zweiten Verstärkungspunkt. Erfindungsgemäß sind die Verstärkungspunkte jeweils an einem Übergang zwischen Nasenrückenbereich zu Seitenbereich angeordnet. Optional oder zusätzlich erstreckt sich der Bogen von einem Verstärkungspunkt über den Seitenbereich des Maskenwulstes zu einem weiteren Verstärkungspunkt, der an dem Übergang des Seitenbereichs zum Basisbereich des Maskenwulstes ausgebildet ist. Alternativ oder zusätzlich erstreckt sich der Bogen von dem Verstärkungspunkt am Übergang des Seitenbereichs über den Basisbereich des Maskenwulstes zu einem weiteren Verstärkungspunkt, der an dem Übergang des Basisbereichs zum Seitenbereich des Maskenwulstes ausgebildet ist. Die bogenförmige Ausgestaltung der Verbindungsstruktur ermöglicht eine besonders stabile und gleichmäßige Verteilung des Gewichts des Maskenkörpers. Der Bogen verleiht einem Bereich des Maskenwulstes, über den er sich ausbildet, eine Festigkeit und kann daher auch als festigkeitsgebende Struktur bezeichnet werden.

In einer Weiterbildung des erfindungsgemäßen Maskenwulstes, sind die Verstärkungspunkte im Maskenwulst im Bereich des Auflagebereichs oder an dem Auflagebereich anliegend angeordnet. Die Verstärkungspunkte sind in der Regel berührungsfrei zu dem Auflagebereich des Maskenwulstes ausgebildet. Durch die berührungsfreie Ausgestaltung liegt die Stützstruktur nicht direkt an das Gesicht des Patienten an, wodurch die Bereiche, die direkt mit dem Patienten in Berührung kommen, mit einem weicheren bzw. dünneren Material versehen werden können. Alternativ können die Verstärkungspunkte der Stützstruktur an den Auflagebereich anliegend bzw. im Auflagebereich ausgebildet sein.

In einer weiteren Weiterbildung ist ausgehend von mindestens einer Verbindungsstruktur der Stützstruktur ein Abstützelement ausgebildet, dass sich in Richtung des Maskenwulstanschlusses aufweitet. Das Abstützelement ist eingerichtet, den durch das Gewicht des Maskenwulstes auf die Stützstruktur wirkenden Druck im angelegten Zustand des Maskenwulstes abzustützen. Somit wirkt das Abstützelement zusammen mit der Stützstruktur stabilisierend für den Maskenwulst. Das Abstützelement geht in der Regel von der Verbindungsstruktur, die beispielsweise als Bogen ausgebildet ist, ab. Das Abstützelement kann eine sanduhrförmige Struktur aufweisen. Das Abstützelement kann als Abstandselement ausgebildet sein, welches eingerichtet ist, einen Abstand zwischen der Stützstruktur und dem Maskenwulstanschluss bereitzustellen.

In Ausgestaltung ist das Abstützelement mittig oder seitlich von der Verbindungstruktur ausgehend ausgebildet. Bei einer mittigen Anordnung des Abstützelements erfolgt eine gleichmäßige Ableitung eines Drucks der Stützstruktur. Eine mittige Anordnung des Abstützelements ermöglicht zudem die Ausbildung gleich großer Einstellbereiche. Die Einstellbereiche können u-förmige oder v-förmige Knickbereiche aufweisen. Die mittige Anordnung des Abstützelements ist besonders stabil, wobei der Einstellbereich sowohl in Richtung des Nasenrückenbereichs als auch in Richtung des Basisbereichs gleichmäßig einstellbar bzw. kippbar in Bezug zu einer Senkrechten des Maskenwulstes in angelegtem Zustand ausgebildet ist. Dies bietet den Vorteil, dass der Maskenwulst an eine Gesichtskontur des Patienten, insbesondere eine Nasenrückenform des Patienten, anpassbar ist.

Eine seitliche Anordnung des Abstützelements ermöglicht die Ausbildung zweier unterschiedlich großer Einstellbereiche. Die Einstellbereiche können u-förmige oder v-förmige Knickbereiche aufweisen. Die seitliche Anordnung des Abstützelements ermöglicht somit eine Ausbildung eines ersten, größeren Einstellbereichs und eines zweiten, kleineren Einstellbereichs, wobei der erste Einstellbereich vorzugsweise im Bereich des Nasenrückenbereichs ausgebildet und der zweite Einstellbereich vorzugsweise im Bereich des Basisbereichs ausgebildet ist. Durch die Ausgestaltung zweier unterschiedlich großer Einstellbereiche kann der Maskenwulst entlang der Senkrecht des Maskenwulstes in angelegtem Zustand unterschiedlich stark gekippt werden. Dies bietet den Vorteil, dass durch den größeren Einstellbereich im Bereich des Nasenrückenbereichs der Maskenwulst in diesem Bereich stärker kippbar ist und damit an eine größere Anzahl an verschiedenen Gesichtskonturen bzw. Nasenrückenkonturen, anpassbar ist.

In einer erfindungsgemäßen Weiterbildung ist an mindestens einem Übergang der Stützstruktur zu dem Abstützelement mindestens ein Knickbereich ausgebildet. Der Knickbereich kann u-förmig oder v-förmig ausgebildet sein. Der Knickbereich ist vorzugsweise an einem Einstellbereich ausgebildet, sodass durch die Bewegung des Knickbereichs die Einstellung des Einstellbereichs erfolgt. Ein Knicken bzw. Zusammendrücken des Knickbereichs erfolgt zumeist durch das Aufsetzen, Befestigen und Einstellen des Maskenwulstes an dem Patientengesicht. Bei dem Knicken des Knickbereichs wird der Einstellbereich derart zusammengedrückt, dass dieser sich zu der Außenseite des Maskenwulstes hin wölbt. Je größer der Einstellbereich und je größer der Druck, desto größer kann die Wölbung ausfallen. Der Knickbereich kann durch eine Verringerung der Wandstärke der Stützstruktur oder des Abstützelements ausgebildet sein. Alternativ kann der Knickbereich durch eine Federstruktur ausgebildet sein. Alternativ kann der Knickbereich mittels eines weicheren Materials im Vergleich zu dem Material der Stützstruktur oder des Abstützelements ausgebildet sein.

In einer weiteren Weiterbildung ist mindestens einer der Knickbereiche durch eine Verringerung der Stärke der Stützstruktur ausgebildet. Durch die Verringerung der Wandstärke der Stützstruktur wird ein v-förmige ausgebildeter Knickbereich ausgebildet. Besonders vorteilhaft ist dabei die Ausgestaltung mit einem seitlichen Knickbereich, durch ein seitlich angeordnetes Abstützelement. Alternativ kann mindestens einer der Knickbereiche durch eine Verringerung der Breite der Stützstruktur ausgebildet sein.

In Ausgestaltung bilden die Stützstruktur und mindestens ein Knickbereich mindestens einen Einstellbereich aus. Der Einstellbereich weist in der Regel eine Wandstärke zwischen 0,2 und 0,7 mm auf, insbesondere zwischen 0,3 und 0,5 mm. Der Knickbereich wird vorzugsweise durch die Stützstruktur und das Abstützelement ausgebildet. Durch die Beweglichkeit des Knickbereichs wird der einstellbare Einstellbereich ausgebildet. Je stärker der Knickbereich ausgebildet ist, desto größer kann der Einstellbereich eingestellt bzw. gekippt werden.

Erfindungsgemäß sind die Verstärkungspunkte am Übergang des Nasenrückenbereichs zum Seitenbereich und in einer weiteren Ausgestaltung sind Verstärkungspunkte am Übergang des Basisbereichs zum Seitenbereich ausgebildet. Alternativ sind die Verstärkungspunkte im Nasenrückenbereich, im Seitenbereich oder im Basisbereich angeordnet. In der Regel umfasst der Maskenwulst zwei bis acht Verstärkungspunkte, vorzugsweise vier Verstärkungspunkte.

In Ausgestaltung sind die Verstärkungspunkte der Stützstruktur in mindestens einem Seitenbereich angeordnet.

In weiterer Ausgestaltung ist mindestens eine Verbindungstruktur über den Nasenrückenbereich des Maskenwulstes ausgebildet, wobei die die Verbindungstruktur begrenzenden Verstärkungspunkte an die einem Seitenbereich des Maskenwulstes, separiert durch die Öffnung, angeordnet sind. Die Anordnung der Verstärkungspunkte jeweils seitlich der Öffnung bietet den Vorteil, dass eine stabile Stützstruktur über den Nasenrückenbereich ausgebildet werden kann.

In einer weiteren Weiterbildung der Erfindung ist mindestens eine Verbindungstruktur über einem Seitenbereich des Maskenwulstes ausgebildet, wobei die die Verbindungstruktur begrenzenden Verstärkungspunkte auf einem gemeinsamen Seitenbereich des Maskenwulstes angeordnet sind. Die Anordnung der Verstärkungspunkte auf einer gemeinsamen Seite bzw. einem gemeinsamen Seitenbereich bietet den Vorteil, dass eine stabile Stützstruktur über einen unempfindlichen Seitenbereich ausgebildet werden kann.

In Ausgestaltung weist die Stützstruktur jeweils eine 1/12 bis 1/8, insbesondere eine 1/10 stärkere Wandstärke auf, als der jeweilige Bereich, in dem sie ausgebildet ist. Die Stützstruktur kann dabei unterschiedliche Wandstärken aufweisen. Optional weist die Stützstruktur in einem Teil der Stützstruktur, der benachbart zu dem Nasenrückenbereich ausgebildet ist, eine geringere Wandstärke auf, als in einem Teil der Stützstruktur, der benachbart zu dem Basisbereich ausgebildet ist.

In einer Weiterbildung weist die Stützstruktur eine Wandstärke zwischen 2 mm und 1,8 mm auf und die umgebende Maskenwulstwand weist eine Wandstärke von 1,6 mm auf. Dabei weist die Stützstruktur in dem Teil der Stützstruktur, der benachbart zu dem Nasenrückenbereich ausgebildet ist, eine Wandstärke von 1,8 mm auf und in dem Teil der Stützstruktur, der benachbart zu dem Basisbereich ausgebildet ist, eine Wandstärke von 2 mm.

Erfindungsgemäß weist die Stützstruktur mindestens zwei Verstärkungspunkte und mindestens eine Verbindungsstruktur zwischen zwei Verstärkungspunkten auf, wobei die mindestens eine Verbindungsstruktur als Bogen ausgebildet ist, wobei der Bogen von dem Auflagebereich fortweisend ausgebildet ist.

In einer Weiterbildung ist an mindestens einem Übergang der Stützstruktur zu dem Abstützelement mindestens ein Knickbereich ausgebildet, wobei die Stützstruktur und der mindestens eine Knickbereich mindestens einen Einstellbereich ausbilden, wobei der Einstellbereich eine Wandstärke zwischen 0,2 mm und 0,7 mm, insbesondere zwischen 0,3 mm und 0,5 mm aufweist.

Die vorliegende Erfindung betrifft ferner einen Maskenwulst für ein Patienteninterface bestehend aus einem Nasenrückenbereich, mindestens einem Seitenbereich, einem Basisbereich, einem Maskenwulstanschluss und einem Auflagebereich zum Patienten, wobei der Auflagebereich sich ausgehend von den Seitenbereichen und dem Basisbereich sowie dem Nasenrückenbereich zur Öffnung hin erstreckt, wobei der Auflagebereich als Dichtlippe ausgeführt ist, welche die zentrale Öffnung umrandet, die zumindest der Einführung einer Nase des Patienten dient.

Erfindungsgemäß umfasst der Maskenwulst mindestens eine Stützstruktur und mindestens einen Knickbereich, wobei die Stützstruktur und der mindestens eine Knickbereich mindestens einen Einstellbereich ausbilden, wobei der Einstellbereich eine Wandstärke zwischen 0,2 und 0,7, insbesondere zwischen 0,3 und 0,5 mm aufweist.

Die vorliegende Erfindung betrifft des Weiteren einen Maskenwulst für ein Patienteninterface bestehend aus einem Nasenrückenbereich, mindestens einem Seitenbereich, einem Basisbereich, einem Maskenwulstanschluss und einem Auflagebereich zum Patienten, wobei der Auflagebereich sich ausgehend von den Seitenbereichen und dem Basisbereich sowie dem Nasenrückenbereich zur Öffnung hin erstreckt, wobei der Auflagebereich als Dichtlippe ausgeführt ist, welche die zentrale Öffnung umrandet, die zumindest der Einführung einer Nase des Patienten dient.

Erfindungsgemäß umfasst der Maskenwulst mindestens eine Stützstruktur, wobei die Stützstruktur eine Wandstärke zwischen 2 mm und 1,8 mm aufweist, der Nasenrückenbereich eine Wandstärke von 0,2 - 0,7 mm, insbesondere 0,35 mm aufweist, der Basisbereich eine Wandstärke von 0,5 - 1,2 mm, insbesondere 0,6 mm aufweist und der Seitenbereich eine Wandstärke von 1,2 - 2,5 mm, insbesondere von 1,6 mm aufweist.

Im Folgenden werden anhand von stark vereinfachten schematischen Darstellungen bevorzugte Ausführungsbeispiele der Erfindung näher erläutert. Es zeigt:

Fig.l eine perspektivische Seitenansicht eines erfindungsgemäßen Maskenwulstes mit einer -nicht gezeigten- Stützstruktur.

Fig. 2 eine schematische Draufsicht auf den erfindungsgemäßen Maskenwulst mit einer Darstellung eines Nasenrückenbereichs, zwei Seitenbereichen und einem Basisbereich,

Fig. 3 zeigt eine Seitenansicht des erfindungsgemäßen Maskenwulstes,

Fig. 4 zeigt einen Längsschnitt des erfindungsgemäßen Maskenwulstes mit einer Stützstruktur und einem Abstützelement,

Fig. 5a zeigt eine Seitenansicht auf eine weitere Ausführungsform des erfindungsgemäßen Maskenwulstes mit der -nicht sichtbaren - Stützstruktur, dem -nicht sichtbaren -Abstützelement, zwei Einstellbereichen und zwei Knickbereichen,

Fig. 5b zeigt einen Längsschnitt durch die in der Figur 5a gezeigten Ausführungsform des erfindungsgemäßen Maskenwulstes mit der Stützstruktur, dem Abstützelement, zwei Knickbereichen und zwei Einstellbereichen,

Fig. 6a zeigt eine weitere alternative Ausführungsform des erfindungsgemäßen Maskenwulstes mit der -nicht gezeigten- Stützstruktur, dem -nicht gezeigten-Abstützelement, zwei Knickbereichen und zwei Einstellbereichen,

Fig. 6b zeigt einen Längsschnitt durch die in der Figur 6a gezeigten Ausführungsform des erfindungsgemäßen Maskenwulstes mit der Stützstruktur, dem Abstützelement, zwei Einstellbereichen sowie zwei Knickbereichen in nicht genicktem Zustand,

Fig. 6c zeigt einen Längsschnitt durch die in der Figur 6a und 6b gezeigten Ausführungsform des erfindungsgemäßen Maskenwulstes mit der Stützstruktur, dem Abstützelement, zwei Einstellbereichen sowie zwei Knickbereichen in genicktem Zustand,

Fig. 7 zeigt einen Längsschnitt durch eine Ausführungsform eines erfindungsgemäßen Maskenwulstes,

Fig. 8 zeigt eine perspektivische Ansicht auf eine Öffnung des erfindungsgemäßen Maskenwulstes,

Fig. 9 zeigt eine perspektivische Ansicht auf die Öffnung des erfindungsgemäßen Maskenwulstes mit einem Auflagebereich.

In den Figuren weisen dieselben konstruktiven Elemente jeweils dieselben Bezugsziffern auf.

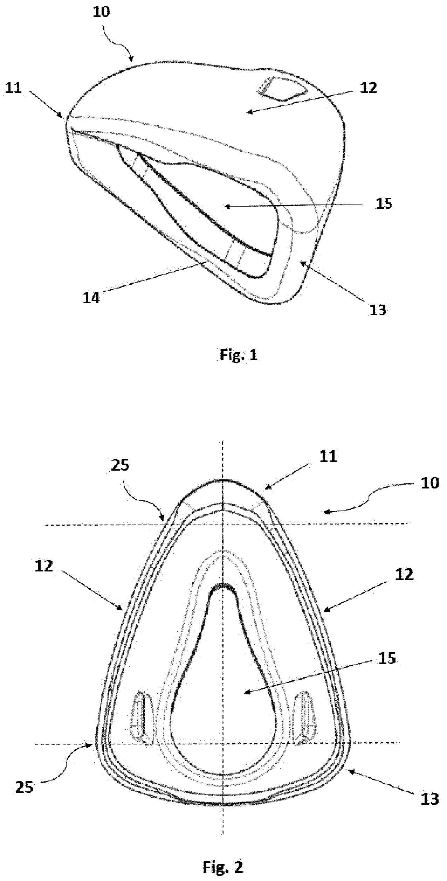

Figur 1 zeigt eine perspektivische Seitenansicht eines erfindungsgemäßen Maskenwulstes 10 mit einer - nicht gezeigten - Stützstruktur. Dabei sind ein, eine Öffnung 15 umgebender Auflagebereich 14, ein Nasenrückenbereich 11, ein Seitenbereich 12 sowie ein Basisbereich 13 des Maskenwulstes 10 dargestellt. Der Auflagebereich 14 ist als Dichtlippe ausgebildet und umrandet die gesamte Öffnung 15. Der Maskenwulst weist im Nasenrückenbereich 11, im Seitenbereich 12 und im Basisbereich 13 unterschiedliche Wandstärken auf.

Figur 2 zeigt eine schematische Draufsicht auf den erfindungsgemäßen Maskenwulst 10 mit einer Darstellung eines Nasenrückenbereichs 11, zwei Seitenbereichen 12 und einem Basisbereich 13 des Maskenwulstes 10. Der Nasenrückenbereich 11 ist ein Bereich, der sich gegenüberliegend dem Basisbereich 13 befindet. Zwischen dem Nasenrückenbereich 11 und dem Basisbereich 13 sind separiert durch die Öffnung 15 jeweils die beiden Seitenbereiche 12 angeordnet. Die Öffnung 15 ist eingerichtet, zumindest teilweise eine Nase eines Patienten aufzunehmen.

Der Nasenrückenbereich 11 ist ein Bereich des Maskenwulstes 10, in dem dieser eine Wölbung aufweist, die einen Winkel >180° aufweist. Der Basisbereich 13 ist ein Bereich des Maskenwulstes 10, der dem Nasenrückenbereich 11 gegenüberliegend ausgebildet ist und eine im Wesentlichen lineare Ausprägung aufweist. Der Seitenbereich 12 ist ein Bereich des Maskenwulstes 10, der sich zwischen dem Nasenrückenbereich 11 und dem Basisbereich 13 erstreckt. Der Maskenwulst 10 weist somit zwei Seitenbereiche 12 auf, die separiert durch die Öffnung 15 gegenüberliegend zueinander angeordnet sind. Die beiden Seitenbereiche 12 weisen zueinander eine konische Form auf.

Die verschiedenen Bereiche (Nasenrückenbereich 11, Seitenbereiche 12 und Basisbereich 13) sind durch Übergangsbereiche 25 miteinander verbunden. Der Übergangsbereich 25 zwischen dem Nasenrückenbereich 11 und einem Seitenbereich 12 ist der Bereich, an dem der Winkel des Maskenwulstes 10 von einer gebogenen Form in eine lineare Form übergeht. Der Übergangsbereich 25 zwischen Basisbereich und Seitenbereich ist der Bereich in dem der Maskenwulst von der konisch verlaufenden Form in einem Winkel von über 90° in einen linearen Verlauf übergeht.

Insgesamt sind in der vorliegenden Ausführungsform der Nasenrückenbereich 11, die Seitenbereich 12 und der Basisbereich 13 in einem ungefähren Größenverhältnis von 1:1:3 ausgebildet. Optional können die Größenverhältnisse anders gewählt sein.

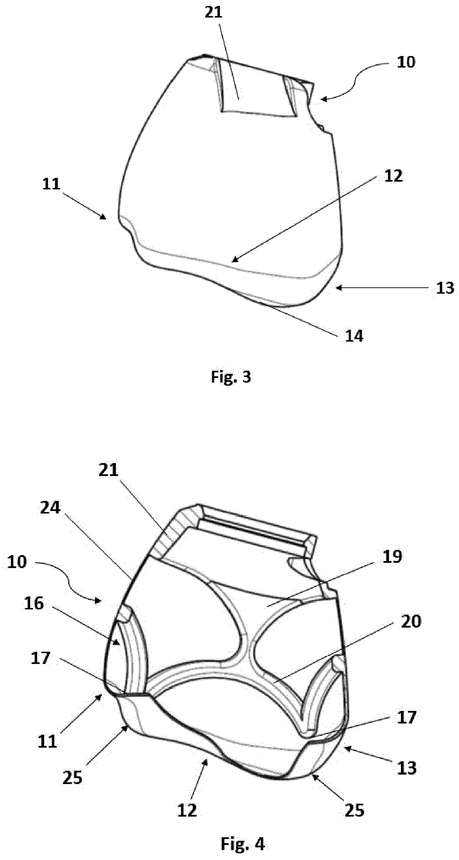

Figur 3 zeigt eine Seitenansicht des erfindungsgemäßen Maskenwulstes 10. Gezeigt ist der Nasenrückenbereich 11, der Seitenbereich 12, der Basisbereich 13 und der Auflagebereich 14. Der Auflagebereich 14 erstreckt sich umlaufend um eine - nicht gezeigte - Öffnung des Maskenwulstes 10. Gegenüber dem Auflagebereich 14 ist ein Maskenwulstanschluss 21 ausgebildet, der eingerichtet ist, den Maskenwulst 10 an ein Schlauchsystem oder beispielsweise eine Stirnstütze anzuschließen.

Figur 4 zeigt einen axialen Schnitt in Längsrichtung des in den Figuren 1 bis 3 gezeigten erfindungsgemäßen Maskenwulstes 10 mit einer Stützstruktur 16 und einem Abstützelement 19. Der Längsschnitt zeigt somit eine Innenansicht des Maskenwulstes 10 in angelegtem Zustand in senkrechter Richtung. Dargestellt ist der Maskenwulst 10 mit der Maskenwulstwand 24, dem Nasenrückenbereich 11, dem Seitenbereich 12, dem Basisbereich 13 und dem Maskenwulstanschluss 21.

In der vorliegenden Ausführungsform des erfindungsgemäßen Maskenwulstes 10 umfasst die Stützstruktur 16 mindestens zwei Verstärkungspunkte 17, die jeweils in einem Übergangsbereich 25 des Nasenrückenbereichs 11 zum Seitenbereich 12 bzw. des Seitenbereichs 12 zum Basisbereich 13 angeordnet sind, sowie eine als Bogen ausgeformte Verbindungsstruktur 20, die zwischen den Verstärkungspunkten 17 angeordnet ist. Die Stützstruktur 16 ist berührungsfrei zu dem Auflagebereich 14 ausgebildet. Die Stützstruktur 16 ist in die Maskenwulstwand 24 integriert ausgebildet. Sie weist eine stärkere Wandstärke auf, als die sie umgehende Maskenwulstwand 24 in den einzelnen Bereichen (Nasenrückenbereich 11, Seitenbereiche 12, Basisbereich 13). In der Regel weist die Stützstruktur eine $\frac{1}{10}$ stärkere Wandstärke auf, als die sie umgebende Maskenwulstwand 24. Die Stützstruktur 16 ist als ein verdickter Bogenstrang ausgebildet, der integral in dem Maskenwulst 10 ausgebildet ist. Die Stützstruktur 16 kann rundlich, oval oder kantig ausgebildet sein.

In der Figur 4 ist ferner ein Abstützelement 19 dargestellt, welches im Bereich eines Scheitelpunktes der Verbindungsstruktur 20 angeordnet ist und sich ausgehend von der Stützstruktur 16 sanduhrförmig in Richtung des Maskenwulstanschlusses 21 erstreckt. In der vorliegenden Ausführungsform ist das Abstützelement 19 in die Maskenwulstwand 24 integriert ausgebildet. Das Abstützelement 19 weist in der Regel die gleiche Wandstärke wie die Stützstruktur 16 auf. Die Wandstärke des Abstützelements 19 kann sich jedoch auch von der Wandstärke der Stützstruktur 16 unterscheiden.

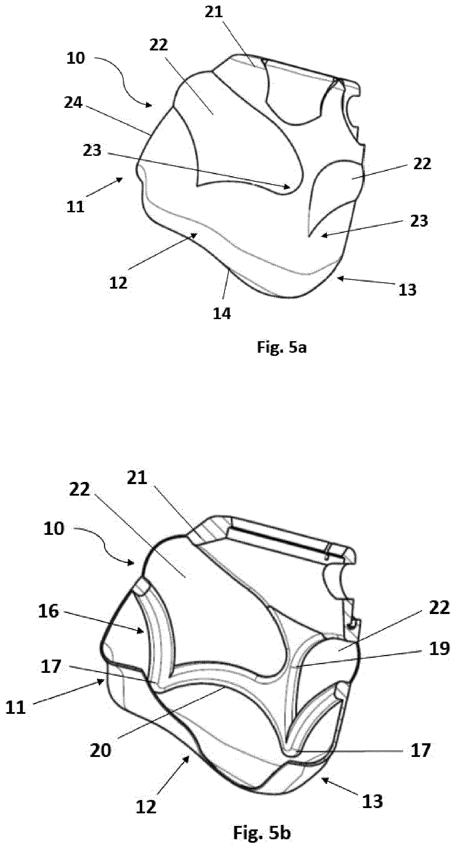

In der Figur 5a ist eine perspektivische Seitenansicht des erfindungsgemäßen Maskenwulstes 10 dargestellt, wobei die Maskenwulstwand 24, der Nasenrückenbereich 11, der Seitenbereich 12, der Basisbereich 13 sowie der Auflagebereich 14 und der Maskenwulstanschluss 21 gezeigt sind.

Ferner sind Einstellbereiche 22 dargestellt. Die Einstellbereiche 22 werden durch die - nicht gezeigte - Stützstruktur und mindestens ein - nicht gezeigtes - Abstützelement gebildet. Die Einstellbereiche 22 weisen Knickbereiche 23 auf, die u- förmig oder v-förmig ausgebildet sind.

Die Einstellbereiche 22 sind in der Maskenwulstwand 24 integriert ausgebildet und weisen dieselbe Wandstärke auf, wie die Maskenwulstwand 24. Sie können in einer weiteren Ausführungsform jedoch auch unterschiedliche Wandstärken aufweisen. Die Einstellbereiche 22 sind eingerichtet, bei einer Druckausübung auf den Maskenwulst 10 eine Wölbung auszubilden, wodurch der Abstand zwischen der -nicht gezeigten - Stützstruktur 16 und dem Maskenwulstanschluss 21 verringert wird. Die Wölbung kann umlaufend um den Maskenwulst im Bereich des Einstellbereichs 22 ausgebildet sein. In der vorliegenden Ausführungsform weisen die Einstellbereiche 22 eine unterschiedliche Ausprägung aus, so gibt es einen ersten, größeren Einstellbereich und einen zweiten kleineren Einstellbereich. Die Ausprägung der Einstellbereiche 22 ist durch die Anordnung des Abstützelements 19 bedingt.

In der Figur 5a ist somit der erfindungsgemäße Maskenwulst 10 dargestellt, wobei in der vorliegenden Ausführungsform die Einstellbereiche 22 durch ein - nicht gezeigtes - seitlich an der Verbindungsstruktur 16 angeordnetes Abstützelement 19 ausgebildet werden. Das Abstützelement 19 gibt zusammen mit der Stützstruktur 16 die Form des Knickbereichs 23 vor.

Figur 5b zeigt einen Längsschnitt durch die in der Figur 5a gezeigten Ausführungsform des erfindungsgemäßen Maskenwulstes 10. Dargestellt sind die gewölbeförmige Struktur 16, die die Verstärkungspunkte 17 und die Verbindungsstruktur 20. Zudem ist das Abstützelement 19 dargestellt, wobei sich das Abstützelement 19 nicht von einem Scheitelpunkt der Verbindungsstruktur 20 ausgehend sanduhrförmig aufweitet, sondern von einem seitlichen Abschnitt der bogenförmig ausgebildeten Verbindungsstruktur 20 ausgebildet ist. Die Einstellbereiche 22 werden durch die Stützstruktur 16, das Abstützelement 22 und den Maskenwulstanschluss 21 begrenzt. Die Einstellbereiche 23 können aus einem elastischeren, dünneren oder anderen Material ausgebildet sein, als die diese umgebenen Bereiche des Maskenwulstes 10. Die Einstellbereich 23 können über interne Versteifungsstrukturen verfügen, die beispielsweise durch unterschiedliche Materialhärten ausgebildet sein können.

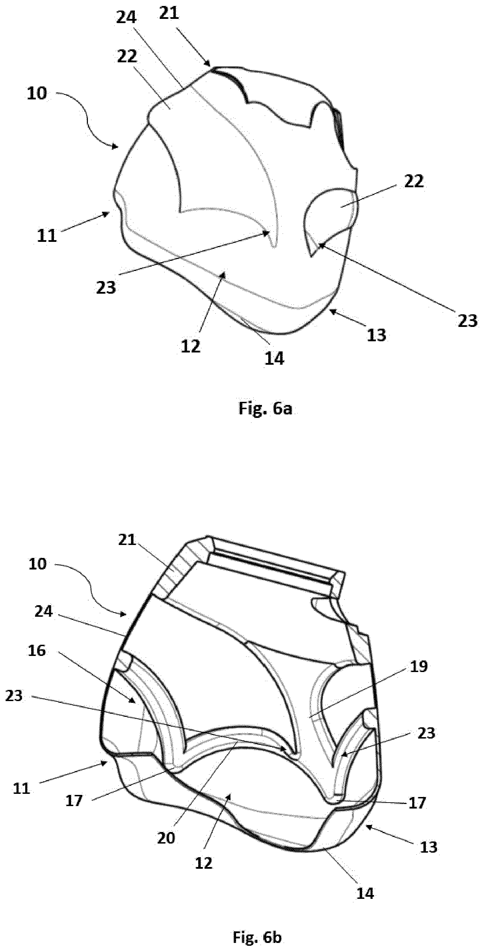

Figur 6a zeigt eine weitere alternative Ausführungsform des erfindungsgemäßen Maskenwulstes 10 mit dem Maskenwulstanschluss 21, der -nicht gezeigten- Stützstruktur, dem - nicht gezeigten - Abstützelement, zwei Knickbereichen 23 und zwei Einstellbereichen 22.

Dargestellt ist eine Ausführungsform mit zwei unterschiedlich großen Einstellbereichen 22, einem ersten und einem zweiten Einstellbereich 22. Der erste und der zweite Einstellbereich 22 werden durch die Lage des - nicht gezeigten -Abstützelements an der - nicht gezeigten -Stützstruktur bestimmt. Der erste und der zweite Einstellbereich 22 sind eingerichtet, unter Druck eine Wölbung der Maskenwulstwand 24 auszubilden. Die Einstellberieche weisen jeweils Knickbereiche 23 auf, die in der vorliegende Ausführungsform v-förmig ausgebildet sind. Optional können die Knickbereiche 23 auch u-förmig ausgebildet sein.

Figur 6b zeigt einen axialen Längsschnitt durch die in der Figur 6a gezeigten Ausführungsform des erfindungsgemäßen Maskenwulstes 10 mit der Stützstruktur 16, dem Abstützelement 19, zwei Einstellbereichen 22 sowie zwei Knickbereichen 23 in nicht genicktem Zustand. Der erfindungsgemäße Maskenwulst 10 weist das Abstützelement 19 auf, welches sich sanduhrförmig von der Stützstruktur 16 in Richtung des Maskenwulstanschlusses 21 erstreckt. Die Stützstruktur 16 weist die Verstärkungspunkte 17 sowie eine bogenförmig ausgestaltete Verbindungsstruktur 20 auf. Am Übergang der Stützstruktur 16 zum Abstützelement 19 wird der Knickbereich 23 ausgebildet. Im Bereich des Knickbereichs 23 weist die Stützstruktur 16 eine Verringerung/Reduzierung der Wandstärke auf. Durch die Reduzierung der Wandstärke der Stützstruktur 16 kann der Knickbereich 23 vergrößert werden, sodass der durch den Knickbereich 23 gebildete Einstellbereich 22 vergrößert wird.

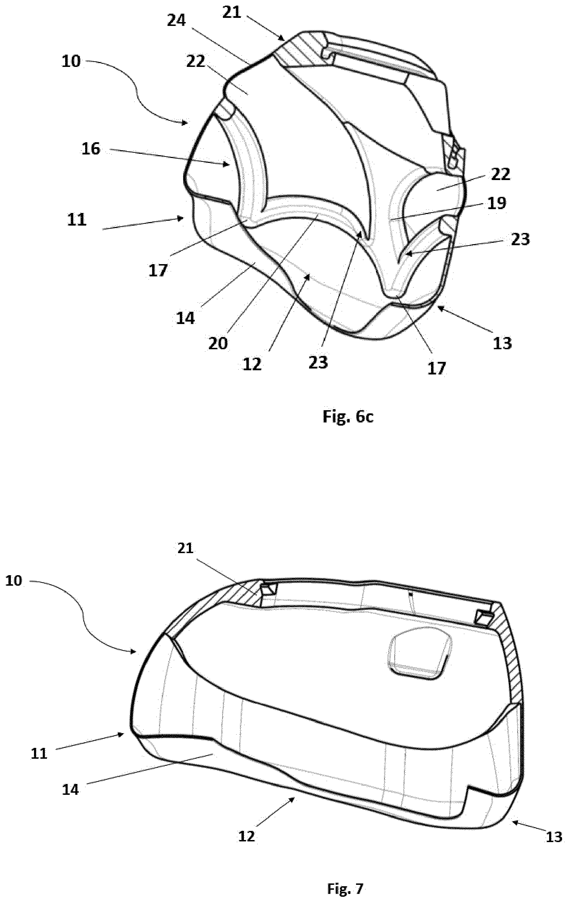

Figur 6c zeigt einen axialen Längsschnitt durch die in der Figur 6a und 6b gezeigten Ausführungsform des erfindungsgemäßen Maskenwulstes 10 mit der Stützstruktur 16, dem Abstützelement 19, Einstellbereichen 22 sowie zwei Knickbereichen 23 in geknicktem Zustand. Dabei weist die Stützstruktur 16 die in Figur 6b beschriebene Reduzierung der Wandstärke auf und das Abstützelement 19 ist seitlich an der Verbindungsstruktur 20 der Stützstruktur 16 angeordnet, wodurch ein erster, großer Einstellbereich und ein zweiter, kleinerer Einstellbereich 22 ausgebildet sind. Gezeigt ist der erfindungsgemäße Maskenwulst 10 in geknicktem Zustand, d.h. auf den Maskenwulst 10 ist in angelegtem Zustand ein Druck beaufschlagt. Gezeigt ist, dass die Maskenwulstwand 24 in diesem geknickten Zustand gewölbt ausgebildet ist. Somit ist der Abstand zwischen der Stützstruktur 16 und dem Maskenwulstanschluss 21 verringert worden, sodass die überschüssige Maskenwulstwand 24 nach außen gewölbt wird.

Figur 7 zeigt einen Längsschnitt durch eine Ausführungsform eines erfindungsgemäßen Maskenwulstes 10. Dabei sind der Nasenrückenbereich 11, der Seitenbereich 12, der Basisbereich 13 sowie der Auflagebereich 14 dargestellt. Gezeigt ist auch die Stützstruktur 16.

In der vorliegenden Ausführungsform weist die Stützstruktur 16 eine Wandstärke zwischen 1,8 mm bis 2 mm auf. Die Wandstärke der Stützstruktur 16 kann dabei variieren. So kann ein Teil der Stützstruktur 16, der benachbart zu dem Nasenrückenbereich 11 ausgebildet ist, eine geringere Wandstärke, beispielsweise 1,6 mm aufweisen, als ein Teil der Stützstruktur 16, mit beispielsweise 2,0 mm, der benachbart zum Basisbereich 13, ausgebildet ist.

Wie in der Figur 7 dargestellt weist der Maskenwulst 10 unterschiedliche Wandstärken auf. So weist der Nasenrückenbereich 11 eine Wandstärke von 0,2 - 0,7 mmm, insbesondere von 0,35 mm auf. Der Seitenbereich 12 weist eine geringere Wandstärke auf, als die ihn umgebenden Stützstrukturen 16. In der vorliegenden Ausführungsform weist der Seitenbereich 12 eine Wandstärke von 1,6 mm auf.

Der Basisbereich 13 weist in der Regel eine stärkere Wandstärke als diesem gegenüberliegen ausgebildeten Nasenrückenbereich 11 auf. Der Basisbereich weist in der Regel eine Wandstärke von 0,5 - 1,2 mm auf. In der vorliegenden Ausführungsform weist der Basisbereich 13 eine Wandstärke von 0,6 mm auf.

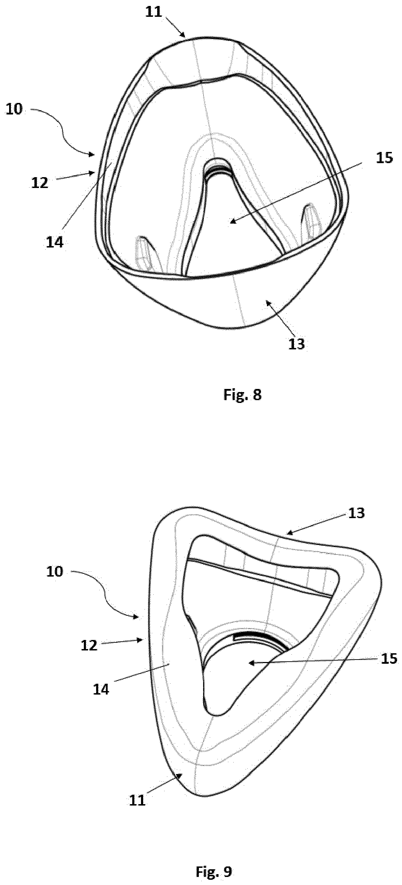

Figur 8 zeigt eine perspektivische Ansicht auf die Öffnung 15 des - in Fig. 7 gezeigten - erfindungsgemäßen Maskenwulstes 10. Dargestellt ist der Nasenrückenbereich 11, sowie die Seitenbereiche 12 und der Basisbereich 13 sowie der Auflagebereich 14. Der Maskenwulst 10 weist eine unterschiedliche Wandstärke auf, wobei die Differenz zwischen der Wandstärke der Stützstruktur 16 und der diese umgebenden Maskenwulstwand 24 gering ist, insbesondere zwischen 0,2 mm und 0,4 mm beträgt.

Figur 9 zeigt eine perspektivische Ansicht auf die Öffnung 15 des erfindungsgemäßen Maskenwulstes 10 mit dem Auflagebereich 14. Dargestellt sind zudem der Nasenrückenbereich 11, die Seitenbereiche 12 sowie der Basisbereich 13, die um die Öffnung 15 angeordnet sind. Der Auflagebereich 14 erstreckt sich umlaufend um die Öffnung 15 und ist als Dichtlippe ausgebildet.

**Bezugszeichenliste**

| 10 | Maskenwulst |
|----|-------------|
| 11 | Nasenrückenbereich |
| 12 | Seitenbereich |
| 13 | Basisbereich |
| 14 | Auflagebereich |
| 15 | Öffnung |
| 16 | Stützstruktur |
| 17 | Verstärkungspunkte |
| 18 | Verbindungsstruktur |
| 19 | Abstützelement |
| 20 | Bogen |
| 21 | Maskenwulstanschluss |
| 22 | Einstellbereich |
| 23 | Knickbereich |
| 24 | Maskenwulstwand |
| 25 | Übergangsbereich |

**Patentansprüche**

1. Maskenwulst (10) für ein Patienteninterface bestehend aus einem Nasenrückenbereich (11), mindestens einem Seitenbereich (12), einem Basisbereich (13), einem Maskenwulstanschluss (21) und einem Auflagebereich (14) zum Patienten, wobei der Auflagebereich (14) sich ausgehend von den Seitenbereichen (12) und dem Basisbereich (13) sowie dem Nasenrückenbereich (11) zur Öffnung (15) hin erstreckt, wobei der Auflagebereich (14) als Dichtlippe ausgeführt ist, welche die zentrale Öffnung (15) umrandet, die zumindest der Einführung einer Nase des Patienten dient, wobei der Maskenwulst (10) aus mindestens einem elastischen Material ausgebildet ist und mindestens eine Stützstruktur (16) umfasst, wobei die Stützstruktur (16) in dem Maskenwulst (10) bereichsweise zum Auflagebereich (14) weisend und bereichsweise zum Maskenwulstanschluss (21) weisend ausgebildet ist, **dadurch gekennzeichnet, dass** die Stützstruktur (16) mindestens zwei Verstärkungspunkte (17) und mindestens eine Verbindungsstruktur (18) zwischen zwei Verstärkungspunkten (17) aufweist, wobei die mindestens eine Verbindungsstruktur (18) als Bogen (20) ausgebildet ist, wobei der Bogen (20) von dem Auflagebereich (14) fortweisend ausgebildet ist, **dadurch gekennzeichnet, dass** die Verstärkungspunkte (17) am Übergang des Nasenrückenbereichs (11) zum Seitenbereich (12) ausgebildet sind.

2. Maskenwulst (10) nach einem der voranstehenden Ansprüche, **dadurch gekennzeichnet, dass** die Stützstruktur (16) die Öffnung (15) zumindest zu zwei Drittel oder vollständig umgibt.

3. Maskenwulst (10) nach einem der voranstehenden Ansprüche, **dadurch gekennzeichnet, dass** die Verstärkungspunkte (17) im Maskenwulst (10) im Bereich des Auflagebereichs (14) oder an dem Auflagebereich (14) anliegend angeordnet sind.

4. Maskenwulst (10) nach einem der voranstehenden Ansprüche, **dadurch gekennzeichnet, dass** ausgehend von mindestens einer Verbindungsstruktur (18) der Stützstruktur (16) ein Abstützelement (19) ausgebildet ist, das sich in Richtung eines Maskenwulstanschlusses (21) aufweitet.

5. Maskenwulst (10) nach Anspruch 4, **dadurch gekennzeichnet, dass** das Abstützelement (19) mittig oder seitlich von der Verbindungsstruktur (18) ausgehend ausgebildet ist.

6. Maskenwulst (10) nach einem der voranstehenden Ansprüche 4 oder 5, **dadurch gekennzeichnet, dass** an mindestens einem Übergang der Stützstruktur (16) zu dem Abstützelement (19) mindestens ein Knickbereich (23) ausgebildet ist.

7. Maskenwulst (10) nach Anspruch 6, **dadurch gekennzeichnet, dass** mindestens einer der Knickbereiche (23) durch eine Verringerung der Stärke der Stützstruktur (16) ausgebildet ist.

8. Maskenwulst (10) nach einem der voranstehenden Ansprüche 6 oder 7, **dadurch gekennzeichnet, dass** die Stützstruktur (16) und mindestens ein Knickbereich (23) mindestens einen Einstellbereich (22) ausbilden.

9. Maskenwulst (10) nach einem der voranstehenden Ansprüche, **dadurch gekennzeichnet, dass** die Verstärkungspunkte (17) der Stützstruktur (16) in mindestens einem Seitenbereich (12) angeordnet sind.

10. Maskenwulst (10) nach einem der voranstehenden Ansprüche, **dadurch gekennzeichnet, dass** mindestens eine Verbindungsstruktur (18) über den Nasenrückenbereich (11) des Maskenwulstes (10) ausgebildet ist, wobei die die Verbindungsstruktur (18) begrenzenden Verstärkungspunkte (17) an je einem Seitenbereich (12) des Maskenwulstes (10) separiert durch die Öffnung (15) angeordnet sind.

11. Maskenwulst (10) nach einem der voranstehenden Ansprüche, **dadurch gekennzeichnet, dass** mindestens eine Verbindungsstruktur (18) über einem Seitenbereich (12) des Maskenwulstes (10) ausgebildet ist, wobei die die Verbindungsstruktur (18) begrenzenden Verstärkungspunkte (17) auf einem gemeinsamen Seitenbereich (12) des Maskenwulstes (10) angeordnet sind.

12. Maskenwulst (10) für ein Patienteninterface nach einem der voranstehenden Ansprüche wobei die Stützstruktur (16) eine Wandstärke zwischen 2 mm und 1,8 mm aufweist, der Nasenrückenbereich eine Wandstärke von 0,2 - 0,7 mm, insbesondere 0,35 mm aufweist, der Basisbereich eine Wandstärke von 0,5 - 1,2 mm, insbesondere 0,6 mm aufweist und der Seitenbereich eine Wandstärke von 1,2 - 2,5 mm, insbesondere von 1,6 mm aufweist.

**Claims**

1. A mask bead (10) for a patient interface consisting of a nose bridge region (11), at least one side region (12), a base region (13), a mask bead connection (21) and a bearing region (14) on the patient, wherein the bearing region (14) extends so as to run from the side regions (12) and the base region (13) and the nose bridge region (11) toward the opening (15), wherein the bearing region (14) is designed as a sealing lip which surrounds the central opening (15) which serves at least for the introduction of a nose of the patient, wherein the mask bead (10) is configured from at least one elastic material and comprises at least one supporting structure (16), wherein the supporting structure (16) is configured in the mask bead (10) so as to face partially toward the bearing region (14) and partially toward the mask bead connection (21), **characterized in that** the supporting structure (16) has at least two reinforcing points (17) and at least one connecting structure (18) between two reinforcing points (17), wherein the at least one connecting structure (18) is configured as an arch (20), wherein the arch (20) is configured to face away from the bearing region (14), **characterized in that** the reinforcing points (17) are configured at the transition of the nose bridge region (11) to the side part (12).

2. The mask bead (10) according to one of the preceding claims, **characterized in that** the supporting structure (16) surrounds the opening (15) at least by two thirds or entirely.

3. The mask bead (10) according to one of the preceding claims, **characterized in that** the reinforcing points (17) in the mask bead (10) are arranged in the region of the bearing region (14) or so as to bear against the bearing region (14).

4. The mask bead (10) according to one of the preceding claims, **characterized in that** a support element (19) which widens in the direction of a mask bead connection (21) is configured so as to run from at least one connecting structure (18) of the supporting structure (16).

5. The mask bead (10) according to claim 4, **characterized in that** the support element (19) is configured so as to run centrally or laterally from the connecting structure (18).

6. The mask bead (10) according to one of the preceding claims 4 or 5, **characterized in that** at least one kink region (23) is configured at at least one transition of the supporting structure (16) to the support element (19).

7. The mask bead (10) according to claim 6, **characterized in that** at least one of the kink regions (23) is configured by a reduction in the thickness of the supporting structure (16).

8. The mask bead (10) according to one of the preceding claims 6 or 7, **characterized in that** the supporting structure (16) and at least one kink region (23) form at least one adjusting region (22).

9. The mask bead (10) according to one of the preceding claims, **characterized in that** the reinforcing points (17) of the supporting structure (16) are arranged in at least one side region (12).

10. The mask bead (10) according to one of the preceding claims, **characterized in that** at least one connecting structure (18) is configured over the nose bridge region (11) of the mask bead (10), wherein the reinforcing points (17) defining the connecting structure (18) are arranged on each respective side region (12) of the mask bead (10) separated by the opening (15).

11. The mask bead (10) according to one of the preceding claims, **characterized in that** at least one connecting structure (18) is configured over a side region (12) of the mask bead (10), wherein the reinforcing points (17) defining the connecting structure (18) are arranged on a common side region (12) of the mask bead (10).

12. The mask bead (10) for a patient interface according to one of the preceding claims, wherein the supporting structure (16) has a wall thickness of between 2 mm and 1.8 mm, the nose bridge region has a wall thickness of 0.2 - 0.7 mm, in particular 0.35 mm, the base region has a wall thickness of 0.5 - 1.2 mm, in particular 0.6 mm, and the side region has a wall thickness of 1.2 - 2.5 mm, in particular of 1.6 mm.

## Revendications

1. Jupe de masque (10) pour une interface patient, comprenant une région d'arête nasale (11), au moins une région latérale (12), une région de base (13), un raccord de jupe de masque (21) et une région d'appui (14) sur le patient, dans laquelle la région d'appui (14) s'étend à partir des régions latérales (12), de la région de base (13) et de la région d'arête nasale (11) vers l'ouverture (15), dans laquelle la région d'appui (14) est réalisée comme une lèvre d'étanchéité, laquelle entoure l'ouverture centrale (15) servant au moins à l'introduction d'un nez du patient, la jupe de masque (10) étant réalisée à partir d'au moins une matière élastique et comportant au moins une structure de support (16), dans laquelle la structure de support (16) dans la jupe de masque (10) est conçue de manière à être tournée partiellement vers la région d'appui (14) et partiellement vers le raccord de jupe de masque (21), **caractérisée en ce que** la structure de support (16) présente au moins deux points de renforcement (17) et au moins une structure de renforcement (18) entre deux points de renforcement (17), dans laquelle l'au moins une structure de liaison (18) est conçue comme un arc (20), dans laquelle l'arc (20) est conçu de manière à faire saillie sur la région d'appui (14), **caractérisée en ce que** les points de renforcement (17) sont réalisés au niveau de la transition de la région d'arête nasale (11) vers la partie latérale (12).

2. Jupe de masque (10) selon l'une des revendications précédentes, **caractérisée en ce que** la structure de support (16) entoure l'ouverture (15) au moins aux deux tiers ou complètement.

3. Jupe de masque (10) selon l'une des revendications précédentes, **caractérisée en ce que** les points de renforcement (17) dans la jupe de masque (10) sont disposés de manière adjacente à la région de la région d'appui (14) ou à la région d'appui (14).

4. Jupe de masque (10) selon l'une des revendications précédentes, **caractérisée en ce qu'**un élément de soutien (19) s'élargissant dans la direction d'un raccord de jupe de masque (21) est réalisé à partir d'au moins une structure de liaison (18) de la structure de support (16).

5. Jupe de masque (10) selon la revendication 4, **caractérisée en ce que** l'élément de soutien (19) est réalisé au centre ou latéralement par rapport à la structure de liaison (18).

6. Jupe de masque (10) selon l'une des revendications précédentes 4 et 5, **caractérisée en ce qu'**au moins une région coudée (23) est réalisée au niveau d'au moins une transition de la structure de support (16) vers l'élément de soutien (19).

7. Jupe de masque (10) selon la revendication 6, **caractérisée en ce que** l'une au moins des régions coudées (23) est réalisée par une réduction de l'épaisseur de la structure de support (16).

8. Jupe de masque (10) selon l'une des revendications précédentes 6 et 7, **caractérisée en ce que** la structure de support (16) et au moins une région coudée (23) forment au moins une région de réglage (22).

9. Jupe de masque (10) selon l'une des revendications précédentes, **caractérisée en ce que** les points de renforcement (17) de la structure de support (16) sont disposés dans au moins une région latérale (12).

10. Jupe de masque (10) selon l'une des revendications précédentes, **caractérisée en ce qu'**au moins une structure de liaison (18) est réalisée par le biais de la région d'arête nasale (11) de la jupe de masque (10), dans laquelle les points de renforcement (17) délimitant la structure de liaison (18) sont disposés respectivement dans une région latérale (12) de la jupe de masque (10), séparés par l'ouverture (15).

11. Jupe de masque (10) selon l'une des revendications précédentes, **caractérisée en ce qu'**au moins une structure de liaison (18) est réalisée par le biais d'une région latérale (12) de la jupe de masque (10), dans laquelle les points de renforcement (17) délimitant la structure de liaison (18) sont disposés dans une région latérale (12) commune de la jupe de masque (10).

**12.** Jupe de masque (10) pour une interface patient selon l'une des revendications précédentes, dans laquelle la structure de support (16) présente une épaisseur de paroi entre 2 mm et 1,8 mm, la région d'arête nasale présente une épaisseur de paroi de 0,2 à 0,7 mm, en particulier de 0,35 mm, la région de base présente une épaisseur de paroi de 0,5 à 1,2 mm, en particulier de 0,6 mm, et la région latérale présente une épaisseur de paroi de 1,2 à 2,5 mm, en particulier de 1,6 mm.

**Fig. 1**

**Fig. 2**

Fig. 3

Fig. 4

**Fig. 5a**

**Fig. 5b**

Fig. 6a

Fig. 6b

**Fig. 6c**

**Fig. 7**

Fig. 8

Fig. 9

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2001062326 A1
- US 20020148472 A1